# EUROPEAN PATENT APPLICATION

(11) **EP 2 135 939 A1**
(43) Date of publication of application: **23.12.2009**
(21) Application number: 08380180.3
(22) Date of filing: 20.06.2008
(51) Int. Cl.: C12N 1/12, C12P 1/00, C12P 5/00, C12P 7/64, C12P 19/04, C12P 21/00

(54) **Process for obtaining a high nutritional value product and/or for transforming it into energy resources**

(71) Applicant: Stroïazzo-Mougin, Bernard A. J., 03560 El Campello (Alicante) (ES)
(72) Inventor: Stroïazzo-Mougin, Bernard A.J., 03560 El Campello (Alicante) (ES); Rodriguez Verdu, Eduardo, 03002 Alicante (ES); Chapuli Fernandez, Eloy, 3560 EL Campello (Alicante) (ES)
(74) Representative: Saez Granero, Francisco Javier

(57) **Abstract**

The present invention relates to a novel process for obtaining a high nutritional value product and/or for transforming it into energy resources which comprises the steps of culturing the "phytoplankton", catalytic production, emptying and separation. Said high nutritional value product is alternatively transformed into electric energy, thermal energy, fuel and/or synthesis gas type energy resources.

## Description

### Technical Field of the Invention

The present invention relates to a (continuous industrial) process for obtaining a high nutritional value product and/or for transforming it into energy resources. Said energy resources are applicable, among others, to obtaining thermal energy, electric energy, fuels (solid, liquid, gas), synthesis gas...

Said process is carried out by means of using a magneto-hydro-photosynthetic "catalyst" in the culture and production steps. An emptying step and a separation step also take place in said process which allow obtaining a high energy value product after a processing step.

The present invention further relates to a concentrate of photosynthetic strains obtained by means of the previous process and to the use thereof in different sectors of the art, for obtaining, as previously mentioned, added value and energy resources.

This novel process has the particularity and advantage of being able to recycle carbon dioxide.

### State of the Art

A huge amount of solar energy irradiates the surface of the Earth, (approximately 15,000 times the daily global consumption). In this sense, the energy from the sun is the major and main energy source of this planet. Nevertheless, the effectiveness of the concentration of energy per unit area at any point of the Earth is very low (only about 1 to 5 kW.h/m²/day). This low effectiveness of energy concentration limits the direct use of solar energy as a primary energy source.

Global warming is currently a problem that is giving rise to the development of new technologies in different fields of the art in order to attempt eradicating or minimize the effects of said warming. The obligation of the economic areas to comply with the objectives imposed by the Kyoto protocol on the reduction of CO₂/SO₂ emissions and the emissions of other gases causing the so-called greenhouse effect is leading countries to search for alternative and renewable fuels to avoid possible tax penalties.

Although the production of solar and wind energy is increasing in some regions, these technologies are very expensive, they are not viable in all climatic areas and do not assure a continuous energy production. In these conditions, biofuels are intended to play an essential role as substitutes for fossil fuels, especially for transport and heating applications.

The production costs of biofuels from terrestrial plants, such as palm and rapeseed oil, have always been a reason for concern. Taking into account the low oil production indices per hectare, huge amounts of resources would be needed for a commercial production to be reached. Land and water are two scarce resources and it is preferable to use them for producing food, which are furthermore more profitable for farmers. Furthermore, intensive fertilization is a form of land and water pollution of the first order. Extensive single crop farming is also one of the main enemies of biodiversity.

A study of the University of Berkeley, Natural Resources Research Vol 14 No. 1 March 2005 pp. 65-72 shows that a terrestrial plant, such as sunflower, requires more energy for its transformation into a fuel than the energy capable of supplying as such, for example, for the production of 1,000 kg of sunflower fuel having a calorific value of 9,000,000 Kcal, 19 million Kcal have to be consumed, which corresponds to a CO₂ emission greater than that emitted by a fossil fuel. In other words, if the emission of a 135 hp (100 kW) car on a 100 km trip making use of a fossil fuel is 20 Kg of CO₂, when a sunflower-based fuel is used, the total combined emission would be 36 Kg of CO₂. However, in the case of a fuel based on phytoplankton fed with the CO₂ of a power station, for example a thermal power station, the amount of CO₂ emitted to the atmosphere is 10 kg. This is due to the fact that the CO₂ captured by the phytoplankton has generated in turn a power of 100 kW, therefore the balance of CO₂ emitted per kW produced is half. In addition, the present invention describes a system in which not all the end product is intended for the production of fuels, but also for the production of a high nutritional value product.

It should be emphasized that when the application of the end product is that of generating electric energy, there is no CO₂ emission to the atmosphere as such CO₂ is introduced again in the system for the production of phytoplankton

In view of that discussed above, with a production of phytoplankton which allows substituting the use of fuels of a petroliferous origin, the advantages from the environmental point of view are evident. According to this, the great challenge lies in achieving a culture which allows massively producing this product. To that end, it is essential to suitably select the reactor to be used.

The reactors that are currently used, either on an industrial or experimental scale, can be classified into two large groups:
- Open reactors
- Closed reactors
   o Horizontal
   o Vertical

### Open reactors

They consist of the use of shallow tanks in which algae, water and nutrients circulate through the circuit by means of a motor-driven system of blades.

The great drawback represented by this system is that, since it is completely open, the algae are susceptible of being contaminated by any organism that is introduced into the tank. Furthermore, the conditions of the system, such as temperature, evaporation of water from the culture medium, supply of CO₂ and luminosity are more difficult to control, the photochemical efficiency being low (high cell densities are not reached); therefore the depth of the tank is of a few millimeters, thus requiring large areas to reach high productions. The group of these factors makes this system not be the most suitable one for the mass cultivation of phytoplankton species.

### Closed reactors

These reactors allow overcoming some of the main obstacles faced by an open system; they allow controlling the variables of the system (CO₂, luminosity, temperature...), there is no contamination, and they require smaller areas of land to reach the same production as in open reactors, especially in the event that the arrangement is vertical (greater volume per unit area). All of this makes the photochemical efficiency of the culture higher.

Due to that mentioned above, different authors have developed systems and processes in which photosynthetic phytoplankton species are cultivated which are exposed to light to generate a growth and multiplication thereof in order to finally obtain a usable product (after several modification steps), as is the case of PCT applications WO2007025145A2, WO2006/020177, WO 03/094598 and W02007144441. However all these systems have the problem that they do not work in a clearly continuous manner, do not have a suitable photochemical efficiency, do not have a recirculation of part of their own obtained products, are laborious systems which in some cases are susceptible to contamination, use solar energy in a two-dimensional manner (they do not take advantage of the verticality of the system of the present invention), and in short, have high associated costs (cleaning, disinfection, evaporation of culture water, are energetically deficient [pumping, extraction...]...), and which indirectly generate contamination. Furthermore, systems or processes such as that described in WO2007025145A2 work according to a horizontal arrangement (they use stirring by means of rollers), the resulting products are not reused (as is done in the present invention with the NOx passing through biofilters to be transformed into nitrates and nitrites which are used as a supply of nutrients to the culture medium). These systems or processes obtain amounts of fuels (about 6000 gallons) lower than the system of the present invention.

In addition, the system described herein has the additional advantage that in the production step a magnetic field is applied which causes an acceleration of said step and, therefore, a substantial increase of the amount of obtained product.

In this same sense, the present invention relates to a novel system for obtaining energy resources by means of a system for capturing sunlight and CO₂ for the reconversion thereof into a continuous energy source using catalysts. To minimize direct or indirect contamination, the system, which will be described below, uses nutrients such as atmospheric CO₂, carbon and nitrogen sources from different industrial sectors. By means of the present invention a total or partial recycling (depending on the application) of the CO₂ is achieved and therefore the net emission can be "zero", since the CO₂ generated can return to the system, thus nourishing the phytoplankton. Furthermore, for the purpose of minimizing water consumption, the water is reused after the separation step which will be described in more detail below.

Furthermore, the system has the advantage that the oxygen generated in one of its steps is reused as a raw material for a subsequent transformation of the obtained product.

Another advantage is that up until now there is no system in which a mass culture of photosynthetic strains is carried out as is done in the present invention.

Thus and by means of the present process, a production is obtained that is considerably higher than that achieved in the previously described processes in which 1.5 g/Uday of product are obtained as a maximum, compared to the concentrations obtained by means of the process described herein which manages to obtain up to 8 g/Uday.

Thus a substantial improvement of the systems that are already known in the state of the art is achieved, either in efficiencies (energetic-economic and productive efficiencies) or in minimization of environmental impact. It is therefore a sustainable system both from the energetic and ecological point of view.

### Description

The present invention relates to a novel (continuous industrial) process for obtaining a high nutritional value product and/or for transforming it into energy resources, which does not cause pollution, which can use its own products (CO₂, nitrogen source [nitrates, nitrites...]) as nutrients. Furthermore, by means of said process a concentrate of photosynthetic strains is obtained which will subsequently be transformed by means of different techniques into thermal energy, electric energy, fuels...

In addition, one of the advantages of the present invention is that it is a continuous process, without stops and which therefore works 24 hours a day.

Therefore a first essential aspect of the present invention relates to a process for obtaining a high nutritional value product and/or for transforming it into energy resources which comprises the steps of:
1. Culturing of photosynthetic species with reversed return which in turn comprises the steps of:
   a. inoculating the aquatic photosynthetic strains;
   b. adding the culture medium;
   c. sterilizing;
   d. adding nutrients;
   e. adjusting the temperature;
   f. adjusting the salinity;
   g. adjusting the pH;
   h. adjusting the pressure; and
   i. generating turbulences.
2. Catalytic production which in turn comprises the steps of:
   a. stirring the culture medium;
   b. attempering;
   c. altering metabolic pathways and accelerating cell proliferation;
   d. catalyzing; and
   e. illuminating.
3. Emptying of the culture medium.
4. Separation which in turn comprises the steps of:
   a. preconcentration;
   b. concentration; and
   c. drying.
(1) Culturing of photosynthetic species with reversed return, in which at least one photosynthetic species is cultured. More than one species can be used in the same culture medium, so that the temperature variable does not affect the system (one species predominates over the other species depending on the temperature); it is thus achieved that there are no production peaks depending on the time of year, assuring the continuity of the process, free from stops for changing the phytoplankton species.

In relation to the species of photosynthetic strains used, these are selected from the group consisting of Chlorophyceae, Bacillariophyceae, Dinophyceae, Cryptophyceae, Chrysophyceae, Haptophyceae, Prasinophyceae, Raphidophyceae, Eustigmatophyceae or a combination thereof.

In addition, the culturing step has the peculiarity of being subjected to a reversed return, to ensure that the time of exposure to light of all the photosynthetic individuals is the same.

The photosynthetic species culturing step comprises the following sub-steps:
- Inoculating the aquatic photosynthetic strains.
- Adding the culture medium.
- Sterilizing.
- Adding nutrients.
- Adjusting the temperature.
- Adjusting the salinity.
- Adjusting the pH.
- Adjusting the pressure.
- Generating turbulences.

Said sub-steps are carried out according to the following methodology:
The starting point is the original strain, which is stored in small transparent containers which can be sterilized in an autoclave. The latter are stored in an incubator between 4 and 12 °C, illuminated by fluorescent 8-watt (W) lamps providing a light intensity of 450 lux. The objective is not to accelerate the growth but to maintain the cultures in good conditions. The cultures are not aerated nor is carbon dioxide introduced.

It is necessary to transfer the strain at monthly intervals to maintain it in a good state and vigorous. To that end, after removing the cotton wool stopper of the flask containing the strains and burning the neck of the flask with a Bunsen burner, a 20 to 50 mL inoculum is transferred to another sterile flask containing the medium previously sterilized in an autoclave. The stopper is inserted after burning the neck of the new flask.

The strain (250 mL or less) continues to be isolated under light and a controlled climate (low temperature) and is only used when it is necessary to inoculate. They are neither aerated nor is carbon dioxide added. The inocula (250 mL to 4 L in volume) grow quickly for a period of 7 to 14 days at higher light intensity and temperatures with a carbon dioxide-enriched air supply. When they are ready, a small proportion of the volume is used to initiate new inocula and the main portion for starting cultures on an intermediate scale (20 L).

Once 20L are available, the sequence is that set forth below:
- 50 L
- 250 L
- 750 L
- 2 m³
- 4 m³
- And so on until reaching the basic productive unit.

In relation to the culture medium, it must be emphasized that fresh, salt, brackish type water or water from waste water from urban waste can be used in the present process.

Furthermore, water that is used as culture medium is subjected to a sterilization process by means of applying ultraviolet and to a selective particle (< 1 µ) filtration.

The sterilization of the culture medium is carried out by means of adding antibiotics and fungicides at a concentration from 100 to 300 mg/mL, preferably from 150 to 250 mg/mL and more preferably at a concentration of 200 mg/mL. The antibiotics used are selected from the group consisting of penicillin and streptomycin or mixture thereof and the fungicides are selected from the group consisting of griseofulvin and nystatin or a mixture thereof.

Once the species is or are seeded, and the culture is freed from any type of possible contamination, the nutrients making the culture grow quickly and efficiently are added. Said nutrient supply is carried out in an exogenous or endogenous manner, i.e., part of the nutrients are supplied externally by different sources and/or internally by means of the feedback of by-products generated in the process, or reusable elements such as the culture medium for example.

The nutrients mainly supply carbon, nitrogen, oxygen, hydrogen and phosphorus.

Compounds supplying carbon are selected from the group consisting of CO, CO₂, CaCO₃ and bicarbonate. The latter can come from the tile industry, ceramic industry, marble industry, thermal power stations, electric power stations, cement factories or a combination thereof at concentrations from 0.1 to 5 grams of carbon per liter of culture.

Compounds with a nitrogen supply are selected from the group consisting of NOx, nitrates, nitrites, ammonium, ammonia or a mixture thereof, at concentrations from 0.1 to 2 grams of nitrogen per liter of culture.

Compounds with an oxygen and hydrogen supply are previously treated water and air at concentrations from 0.1 to 2 grams of oxygen/hydrogen per liter of culture.

Compounds supplying the necessary phosphorus to the culture medium are mainly phosphates at concentrations from 0.01 to 2 grams of phosphorus per liter of culture.

Once the nutrients which are necessary as a feed source for the photosynthetic strains are added, the conditions in which the culture must be are adjusted. In this sense, the culture must be at temperatures between 5 to 45°C, preferably from 20 to 30°C and more preferably from 20 to 25°C.

In relation to the salinity, it must be fixed such that the medium is not toxic for the photosynthetic strains; the salinity range will be comprised between 0 to 80 g/L and preferably between 30 to 40 g/L.

The medium must have a pH from 4 to 11 units, though preferably from 6.5 to 8.5.

The pressure which the culture must be subjected to will be between -1 (vacuum) and 5 atmospheres, preferably at atmospheric pressure.

Once the previous parameters are adjusted, the culture medium is subjected to turbulences. The turbulence acts in a preventive way against fouling, benefits the homogeneity of the system and increases the gas diffusion therein. To generate said turbulences air, N₂, CO₂, CO, NOₓ, gases resulting from a combustion are insufflated, separately or a combination thereof, at concentrations from 0.4 to 600 liters per minute and productive unit, the latter being understood as a culture volume ranging between 0.5 to 100 m³, preferably from 2 to 50 m³. Therefore, it is thus achieved that the culture medium is all constant movement, favoring the three-dimensional capture of light. The shadow effect between the photosynthetic individuals is thus prevented because, since it is a non-static process, it is ensured that they capture natural or artificial light.
(2) The second step of the present process is a catalytic production step. Once the selected photosynthetic strains have been suitably cultured, the parameters that speed up said step and make it more efficient are supplied.

In this sense the catalytic production step comprises the following substeps:
- Stirring the culture medium.
- Attempering.
- Altering metabolic pathways and accelerating cell proliferation.
- Catalyzing.
- Illuminating.

The culture medium stirring step has the same purpose as the turbulence generation step of the culturing step.

Said stirring is carried out by means of pulses by injection of air, N₂, CO₂, CO, NOₓ, gases resulting from a combustion, separately or a combination thereof. The amount that is injected must always be 0.4 to 600 liters per m² and per productive unit.

Another step that must be taken into account for a maximum production is the attemperation step, such that the culture must be maintained in the previously described temperature conditions, thus preventing the loss of yield. This attemperation is carried out by means of using heat exchangers, sleeves, and/or submerging the culture medium underground. Furthermore said attemperation can be carried out by adding cold or hot air allowing the culture to be between temperatures from 5 to 45°C.

Then, in order for the culture to be more productive than usual and the mitosis to be quicker, a metabolic pathway alteration and cell proliferation alteration step is carried out by means of applying a variable and/or constant intensity magnetic field. The latter cause the microenvironments which are created inside the cell membranes or in the hydrophobic areas of certain proteins (enzymes, carriers) restricting the mobility of the biomolecules and reducing the rate of the chemical reactions, to be more susceptible to interacting with the magnetic field. Modifications of the genetic expression, of the metabolic reactions, of the intracellular signaling systems or of the permeability of the biological membranes which, suitably amplified, give rise to modifications of cell functions, are thus generated. Thus, the occurrence of resonance phenomena affecting the passage of ions through the membranes and other biological processes is furthermore achieved.

Thus, in the present process, a variable magnetic field of 0.1 to 100 mT and/or a constant magnetic field of 0.01 to 1 T is applied in the production step.

Simultaneously to all previous sub-steps within the production step, the culture medium is subjected to a catalysis step by means of using a magneto-hydro-photosynthetic catalyst and/or by means of using zeolites, both natural and artificial and at concentrations from 1 mg/L of culture up to 1 kg/L of culture, preferably from 100 up to 150 mg/L.

In this same sense, the process can act as a catalyst, thus accelerating the usual behavior of a phytoplankton culture in its natural habitat. This catalyst, a magneto-hydro-photosynthetic catalyst, by the action of photosynthesis, by the influence of a magnetic field in a liquid medium, generates an acceleration in production, (upon accelerating metabolic pathways).

As in the previous step, the culture medium in the production step must be illuminated in a natural, artificial manner or a combination thereof. The culture is exposed to an illumination of 1 to 10 kW · h / m² / day. Furthermore, the illumination is carried out by light-darkness cycles, the light cycles being from 6 to 24 hours and the darkness cycles being from 0 to 18 hours.

In this same sense, it is known that visible light radiation occupies a small band of the spectrum, going from 400 to 700 nm, and located between the ultraviolet (UV) and the infrared (IR) radiations, forming the so-called photosynthetically active radiation (PAR). It is also called visible radiation (VIS), due to the fact that the human eye, which is a sensitive photo-detector organ, allows seeing this radiation; white light is formed by the joining of all the light radiations with different color: violet, blue, green, yellow, orange and red light. A colored substance or compound absorbs the light with the color complementary to the color that is seen in it, i.e., the color that it shows is that of the non-absorbed light, which is seen reflected or transmitted.

About 300 years ago, the Englishman Isaac Newton (1642-1727) dispersed visible light into a color spectrum; in 1900, Max Planck set forth the theory by which any radiant energy transfer from one object to another involved "discrete" units of energy which he called quanta. Based on these observations, it was concluded that light has wave and particle characteristics. The particles are said to correspond to quanta, photons or discrete energy packets, each one associated to a wavelength. The energy of a photon is inversely but proportional to its wavelength.

A molecule which absorbs light (a photon which collides with matter), absorbs an electron of this molecule every time. When the electron is excited, it is displaced from its basal state or initial position in relation to the nucleus to a distance corresponding to the energy of the photon that it absorbed.

Therefore, in the case at hand, when the light impinges on the system containing the culture medium with the photosynthetic species, a pigment molecule (which has absorbed light) in this situation is excited and this excitation energy is used in photosynthesis.

Chlorophylls and other pigments only last very short periods in the excited state.
The excitation energy is used to cause photosynthesis.

Taking into account that the photosynthesis is a quantum process with an extreme energy transfer efficiency, for it to occur it is necessary for the energy of the excited electrons of several pigments to be transferred to an energy collecting pigment, which is called reaction center. In photosynthesis there are two reaction centers, the photosystem I reaction center and the photosystem II reaction center. The reaction centers consist of a chlorophyll molecule associated to particular proteins and to specific membrane components. Energy is said to migrate from one pigment to another until reaching the Reaction center.

Almost all the absorption occurs in the pigments of the chloroplasts. In the thylakoids, each photon excites an electron of a chlorophyll, a carotene or a xanthophyll.

Therefore, as a whole when the system absorbs light, it causes a forced oscillation of an electron cloud and consequently part of the energy is reflected to the exterior, causing a continuous excitation to the nearby molecules.

The oscillations interfere with one another forming an undulating energy movement or overlapping state, something which is sought in this technology, this movement causing that generation of said overlapping state.

It is thus achieved that the process which is being described is more effective, since the energy emitted by the light is constantly absorbed by the medium and in turn is again emitted, creating a continuous excitation/de-excitation system which makes photosynthesis occur more effectively. Furthermore, as the system is under constant stirring, turbulences being caused, it is ensured that the photosynthetic species present in the medium are in constant movement over all parts of the system, making the likelihood of them being at all the points of the system maximum, such that an almost inexistent shadow effect is achieved.

This production step is carried out for cycles of 6 to 120 hours, preferably of 8 to 12 hours.

Thus and at this point, it is achieved that the culture medium has passed through the following steps:
1) Culturing in which a lag or culture medium adaptation phase has occurred or, in other words, the photosynthetic strains adapt their metabolism to the new environmental conditions (of abundance of nutrients) to be able to initiate the exponential growth.
2) Production in which the following phases have occurred:
   a) Exponential or logarithmic phase: in it, the rate of growth is maximum and the generation time is minimum. During this phase the photosynthetic strains consume the nutrients of the medium at a maximum rate.
   b) Stationary phase: in it, the number of photosynthetic strains does not increase (nor the mass or other parameters of the culture). The cells in stationary phase develop a metabolism different from that of the exponential phase and during it an accumulation and release of secondary metabolites occurs.
      From this point onwards, the following two steps of the process are carried out:
3) The third step or emptying step consists of removing or extracting from 10 up to 100% of the culture per production cycle, preferably 100%.
4) The fourth step of the present process consists of a separation step, in which up to 100% of the culture medium is eliminated. The medium or water eliminated will again be fed back to the system.
   In order to eliminate said medium, the separation step comprises the following sub-steps:
   - Preconcentration.
   - Concentration.
   - Drying.
   The preconcentration step allows eliminating up to 99% of the liquid medium containing the photosynthetic strains. The preconcentration is carried out, and in a non-limiting sense, by means of techniques selected from the group consisting of filtration, flocculation electrocoagulation, ultrasound, mechanical vapor recompression, centrifugation, decantation or a combination thereof.
   Then, after the preconcentration step, a liquid concentrate is obtained, which is subjected to a concentration step for the purpose of eliminating up to 99.5% of the culture medium remaining from the previous step. The concentration is carried out by means of techniques selected from the group consisting of centrifugation, mechanical vapor recompression, decantation, press filter or a combination thereof.
   After this step, a concentrate with a very low percentage of moisture is obtained, which is subjected to a last drying step, from which up to 100% of the remaining liquid medium is eliminated, obtaining an end product with a high energy power and/or added value products.
   The drying is carried out by means of techniques, and in a non-limiting sense, selected from the group consisting of low-temperature thermal drying, turbodryer, spray dryer, lyophilization, multiple effect evaporator, air drying or by a combination thereof.
   Once the drying sub-step has ended, a concentrate of photosynthetic strains with a high nutritional value is obtained.
   According to a preferred embodiment, after the separation step and after obtaining the end product, the latter is subjected to a transformation step giving rise to the fact that said product is the "raw material" for obtaining sources of thermal energy, electric energy, fuels... and added value resources.
5) The transformation step can therefore follow several routes:
   a. Gasification;
   b. Pyrolysis;
   c. Thermochemical liquefaction;
   d. Hydrogen production;
   e. Extraction and transesterification-esterification;
   f. Anaerobic digestion; and
   g. Direct combustion.

   a. Gasification is a set of thermochemical reactions occurring in an oxygen-poor environment, and which results in the transformation of a solid into a series of gases susceptible of being used in a boiler, in a turbine or in a motor, after being suitably conditioned. In addition the gasification as a process concept can be applied for synthesizing high quality liquid fuels, according to the Fisher Tropsch process.
      Said Fisher Tropsch process involves the following reactions
      The main reactions are:

      *nCO* + (2*n* + 1)*H*₂⇄*CₙH*_{2*n*+2} + *nH*₂*O* (Paraffin production)

      *nCO* + (2*n*)*H*₂⇄*CₙH*_{2*n*} + *nH*₂*O* (Olefins production)

      In both cases they are very exothermic reactions, i.e., reactions releasing a large amount of heat.
      Unwanted, secondary reactions:

      *CO* + 3*H*₂⇄*CH*₄ + *H*₂*O* (Methane production)

      *nCO* + (2*n*)*H*₂⇄*CₙH*_{2*n*+1}*OH* + (*n* - 1)*H*₂*O* (Alcohol production)

      2*CO⇄C*(*s*) + *CO*₂ (Solid carbon deposition)

      The reaction is carried out on cobalt or iron catalysts. For a good yield, high pressure (normally 20 - 30 bar) and temperature (200 - 350°C) are required. Above 400°C, methane formation is excessive.
      The main Fischer-Tropsch reactions are actually polymerization reactions, consisting of five basic steps:
      1. Adsorption of CO on the surface of the catalyst
      2. Initiation of the polymerization by means of methyl radical formation (by dissociation of CO and hydrogenation)
      3. Polymerization by condensation (addition of CO and H₂ and release of water)
      4. Termination
      5. Desorption of the product
      The product obtained at the outlet of a Fischer-Tropsch reactor consists of a mixture of hydrocarbons with a very wide distribution of molecular weights, going from gases to waxes including gasoline, kerosene and gas oil.
      The nature and proportion of the products depend on the type of reactor and catalyst. The processes operating at a high temperature generally produce a majority of olefin gasolines while those at a low temperature mainly give paraffin gas oils.
   b. Pyrolysis for obtaining a liquid mixture of oxygenated organic compounds. The pyrolysis process consists of a chemical decomposition of organic matter, caused by heating in the absence of oxygen or other reagents, except possibly steam. A gas fraction and another solid fraction (char) are obtained as the result of this decomposition. The gaseous fraction in turn has a condensable fraction (mixture of long-chain compounds: bio-oil) and another non-condensable fraction (short-chain compounds, H₂, CH₄, C₂H₄, C₂H₆...).
      The amount of hydrocarbons, and therefore of bio-oil in the event that said fraction is desired, which can be obtained from the starting biomass is directly related to the C and H content; therefore proteins, lipids and carbohydrates (the major components of biomass: approximately involve 90% of the total content) are susceptible to being transformed into bio-oil, the most interesting being lipids and carbohydrates.
      According to that mentioned, different types of pyrolysis can be carried out, depending on the physical conditions in which it is performed. Thus, factors as the heating rate, the residence time, etc, have a great influence on the distribution of (liquid, solid and gas) products which are obtained.
      TYPES OF PYROLYSIS
      Slow pyrolysis
      Pyrolysis aimed at mainly obtaining gases and solid.
      Low-moderated temperature.
      Long residence times of the vapor.
      Fractions that are achieved after this process:
      Liquid → 30% (mainly water)
      Solid → 35%
      Gays → 35%
      Gasification
      Pyrolysis aimed to obtaining gases.
      High temperatures (>800C).
      Long residence times of the vapor.
      Fractions that are achieved after this process:
      Liquid → 5%
      Solid → 10%
      Gays → 85%
      Fast pyrolysis
      Pyrolysis aimed at obtaining bio-oil.
      Moderate temperature → -500 °C
      Residence time of the vapor → Less than 2 s
      Liquid (bio-oil)→ 70%
      Solid → 15%
      Gas → 15%
      The main usefulness of fast pyrolysis lies in that it allows transforming a low density fuel of biological origin, with its consequent high transport cost, into a high density liquid fuel which can be transported and used by means of conventional systems, further having the advantage of being able to be used as a fuel in generation turbines, allowing to take advantage of the high yields of the integrated generation schemes.
      Long residence times can cause the cracking of the primary products, thus reducing the yield and modification of the properties of the bio-oil. Furthermore, a low heating rate and a high residence time involve a higher energy demand.
   c. Thermochemical liquefaction. Like pyrolysis, thermochemical liquefaction consists of a chemical decomposition of organic matter, caused by heating in the absence of oxygen or other reagents, except steam. A gas fraction and another solid fraction (char) are obtained as a result of this decomposition. The gaseous fraction in turn has a condensable fraction (mixture of long-chain compound: bio-oil) and another non-condensable fraction (short-chain compounds, H₂, CH₄, C₂H₄, C₂H6...). The main difference is that this process is carried out at high pressures (in the order of 10 MPa) and low temperatures (in the order of 300°C); these working conditions allow that the starting raw material does not require being dried, as a raw material with a moisture of up to 60% can be introduced in the thermochemical liquefaction reactor, which has the clear advantage of preventing drying (thermal energy saving).
   d. Hydrogen (biohydrogen) production. Hydrogen can be produced by algae under specific conditions. There are three different ways that can give rise to the H2 production: direct or indirect photolysis, ATP-driven, H2-production. Direct photolysis is carried out when the resulting hydrogen and oxygen are continuously extracted. This process involves a high cost and high complexity as it is necessary to separate the oxygen from the hydrogen.
      For that reason, it is preferable work according to indirect processes. In these processes, hydrogen is obtained directly from the alga in anaerobic conditions and in conditions with a sulfur limitation.
   e. Extraction and transesterification-esterification for obtaining esters.
      Biodiesel is a fuel formed approximately by 98% of esters, which allows partially or completely substituting the diesel oil in diesel engines without special conversions, adjustments or regulations of the engine being necessary.
      Esters are obtained from a transesterification reaction wherein the triglycerides react with an alcohol, generally methanol, obtaining as glycerin as a by-product; not only is economic efficiency of biodiesel production obtained, but also of the by-product (glycerin):

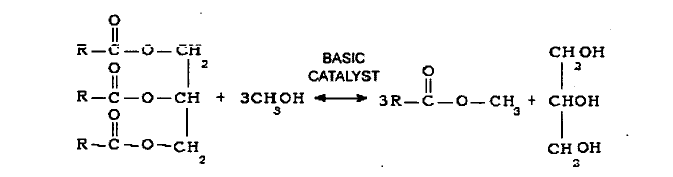

      OIL + ALCOHOL → METHYL ESTER + GLYCERIN

      Catalysts, generally basic catalysts, are used to accelerate the transesterification reaction, which catalysts allow reaching reasonable reaction times at atmospheric pressure and moderate temperatures (about 60°C).
      However, when a basic catalyst is used, it reacts with the fatty acids (triglycerides, diglycerides, monoglycerides, methyl esters and free fatty acids) present in the raw material, giving rise to a saponification reaction. The soaps thus obtained will give rise to an emulsification, which will make it difficult to separate the two products obtained from the transesterification reaction (methyl esters and glycerin).
      According to this situation, whenever the fatty acid content is considerable, an initial phase, prior to the transesterification, which allows reducing the initial concentration of these acids will be necessary. The presence of free fatty acids will have to be especially taken into account since, as will be demonstrated, their saponification will carry the most weight and will therefore produce most emulsification; that is why it is important to take into account their presence in the initial raw material, especially when the content is greater than 0.5%, because below this value the emulsification is not enough to make it difficult to separate the two phases [3].
      Depending on the concentration of free fatty acids in the raw material the performance will vary:
      - If it is a slightly greater than 0.5% the step consists simply of a neutralization or washing of the initial oil with warm water (30-40°C) and small amounts of NaOH (about 1% or less); subsequently the oil is washed with more water to remove all the soaps and it is then dried.
      - If it is considerably greater, this step is called esterification step and consists of reacting the free fatty acids with an alcohol by means of acid catalysis, generally at atmospheric pressure and moderate temperatures; the triglycerides present hardly react with the alcohol, as the rate of reaction in acid medium is very slow, being considered negligible.
         To obtain the triglycerides and free fatty acids present in the microalgae, it will be necessary to carry out an extraction, preferably using suitable solvents for such purpose.
   f. Anaerobic Digestion. Anaerobic digestion is a biological process in which organic matter, in the absence of oxygen, and by means of the action of a group of specific bacteria, is decomposed into gaseous products or "biogas" with the following composition:
      - 50-70% CH₄
      - 30-40% CO₂
      - 5% H₂, H₂S and other gases
         According to this, the biogas produced in anaerobic digestion processes can have different uses:
      - In a boiler for generating heat or electricity
      - In motors or turbines for generating electricity
      - In fuel cells, after a cleaning of H2S and other contaminants of the membranes.
      - Purifying it and add the necessary additives in order to introduce it in a natural gas transport network.
      - Use as a base material for the synthesis of high added value products such as methanol or liquefied natural gas.
      - Automotive fuel.
         Biogas contains a high percentage of methane, CH₄ (between the 50-70%), therefore it is susceptible to taking advantage of the energy by means of its combustion in motors, in turbines or in boilers, either alone or mixed with another fuel.
   g. Direct combustion for generating electricity. The end product with a moisture in the order of 10% is subjected to a combustion process (an exothermic chemical reaction in which a fuel element is combined with another combustion agent, generally oxygen in gaseous form, for generating heat or both, heat and light) such that the calorific value of the gases generated in the process allows producing pressurized vapor, which feeds a steam turbine to obtain electric energy. At this point, cogeneration (a process by means of which useful thermal energy and electric energy can be obtained simultaneously), and the combined cycle (the coexistence of two thermodynamic cycles in one and the same system, a cycle the working fluid of which is steam and another cycle the working fluid of which is a gas product of a combustion) are also taken into account.

According to another preferred embodiment, a feedback step is carried out after the transformation step and in the separation step" by means of which some of the products obtained or the medium containing the photosynthetic strains or the oxygen generated in photosynthesis, are fed back to the culture medium or to other transformation points.

In addition, the gases from the transformation step, are treated with bacteria biofilters such that they are transformed into nitrates and can thus be reconducted to the culturing step as nutrients. Said process is carried out according to the following reactions:

NH₃ + 3/2 O₂ + HCO₃⁻ → NO₂⁻ + CO₂ + 2H⁺ + H₂O (the nitrosomonas bacterium acts)

NO₂⁻ + ½ O₂ → NO₃⁻ (the nitrobacter bacterium acts)

A second essential aspect of the present invention relates to the end product with a high nutritional power value, obtained after the drying step of the present process, which can be used in the food sector due to the fact that certain algae species, given their characteristic composition (rich in proteins and fatty acids), can be used as a nutritional supplement.

Despite the great biological simplicity, the algae strains used in the present process are capable of synthesizing nutritive substances which are stored in their cytoplasm (the part of the cell surrounding the nucleus of the latter). The product obtained essentially contains the following elements in the following ratios:
- High biological value proteins which are in a percentage from 40 up to 85% by weight in relation to the dry product.
- Carbohydrates, which are in a percentage from 10 to 50% by weight in relation to the dry product.
- B-group vitamins, especially vitamin B1 or thiamine, B2 or riboflavin, B3 or niacin and folates which are in a percentage from 0 to 2% by weight in relation to the dry product. Likewise, the presence of beta-carotene (in the intestine, the organism transforms this substance into vitamin A) is emphasized. The publicity on the spirulina algae emphasizes its high vitamin B12 (a vitamin which is absent in food of a plant origin) content but the amount of this nutrient in its natural state listed on the labels is disorienting because is in an inactive form and the body cannot use it. Only those products from plants enriched in vitamin B12 contain the active form of this vitamin.
- Minerals and trace elements: potassium, calcium, zinc, magnesium, manganese, selenium, iron and phosphorus which are in a percentage from 0 to 6% by weight in relation to the dry product.
- Fats, which are in a percentage from 1 to 10% by weight in relation to the dry product and they are mostly in the form of essential fatty acids forming part of the cell membrane of each and every one of the cells of our body.
- Other substances, as chlorophyll, a substance which has the capacity of favoring digestion, which are in a percentage from 0 to 0.5% by weight in relation to the dry product.

A third essential aspect of the present invention relates to the use of the product obtained in the previously described process as a high nutritional value product, for obtaining thermal energy, electric energy, fuels (solid, liquid or gas) and/or synthesis gas.

A series of examples are described below, which must always be taken into account in an illustrative and non-limiting manner.

### Brief Description of the Drawings

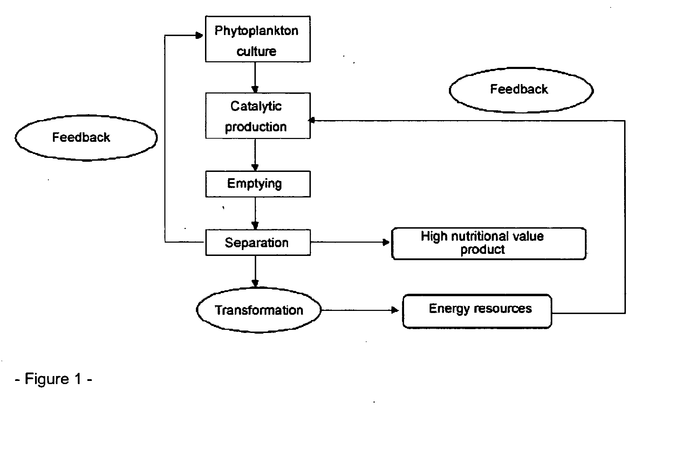

Figure 1 shows a block diagram with each of the steps of the process described in the present invention and the different alternatives thereto, for obtaining a high nutritional value product and/or for transforming it into energy resources.

### EXAMPLES

### 1) PRODUCTION OF ELECTRIC ENERGY (GASIFIER + INTERNAL COMBUSTION ENGINE)

### A) CULTURING AND PRODUCTION

The starting point is a monospecific Nannochloropsis Gaditana culture in brackish well water with a concentration of 4g/L in NaCl.

To start up each of the basic plant systems, the original strain is used as the starting point, which strain is stored in small transparent containers which can be sterilized in an autoclave. The latter are stored in an incubator cooled at 4 to 12 °C, illuminated by fluorescent 8-watt (W) lamps providing a light intensity of 450 lux calculated on the surface of the culture. The objective is not to accelerate the growth but to maintain the cultures in good conditions. The cultures are not aerated nor is carbon dioxide introduced.

It is necessary to transfer the strain at monthly intervals to maintain it in a good state and vigorous. After removing the cotton wool stopper of the flask containing the strains and burning the neck of the flask with a Bunsen burner, a 20 to 50 mL inoculum is transferred to another sterile flask containing the medium previously sterilized in an autoclave. The stopper is inserted after burning the neck of the new flask.

### Steps in the production of algae

The strain (250 mL or less) continues to be isolated under light and a controlled climate (iow temperature) and is only used when it is necessary to inoculate. They are neither aerated nor is carbon dioxide added. The inocula (250 mL to 4 L in volume) grow quickly for a period of 7 to 14 days at higher light intensity and temperatures with a carbon dioxide-enriched air supply. When they are ready, a small proportion of the volume is used to initiate new inocula and the main portion to start cultures on an intermediate scale (20L). Once 20L are available, the sequence is that set forth below:
- 50 L
- 250 L
- 750 L
- 2 m³
- 4.77 m³

The sequence going from 50L up to 4.77 m³ involves a time of 2 weeks. The first system into which CO₂ and air are injected is thus started up. This system allows starting up the remaining systems, as is detailed below:
1. Half of the culture passes to another system which is empty and at that same instant, the necessary water is added to both with the necessary concentration in salt. The latter, which is already attempered, comes from a "surge tank", which has previously been subjected to an ultraviolet treatment (sterilization) and the nutrient will have been added to it (the surge tank has nutrient probes to automate the nutrient supply).
The concentration per liter of culture is that set forth below:

| Component | Stock solution (gL⁻¹ dH₂O) | Amount used | Final concentration |
|---|---|---|---|
| NaNO₃ | 75 | 1 mL | 8.82 x 10⁻⁴ |
| NaH₂PO₄.H₂O | 5 | 1 mL | 3.62 x 10⁻⁵ |
| Na₂SiO₃.9H₂O | 30 | 1 mL | 1.06 x 10⁻⁴ |
| Trace metals | See below | 1 mL | --- |
| Vitamins | See below | 0.5 mL | --- |

Solution of trace metals f/2
Dissolve EDTA and the other components in 950 mL of distilled water. Take to a volume of 1 L of distilled water.

| Component | Stock solution (gL⁻¹ dH₂O) | Amount used | Final concentration |
|---|---|---|---|
| FeCl₃.6H₂O | --- | 3.15 g | 1.17 x 10⁻⁵ |
| Na₂EDTA | --- | 4.36 g | 1.17 x 10⁻⁵ |
| MnCl₂.4H₂O | 180.0 | 1 mL | 9.10 x 10⁻⁷ |
| ZnSO₄.7H₂O | 22.0 | 1 mL | 7.65 x 10⁻⁸ |
| CoCl₂.6H₂O | 10.0 | 1 mL | 4.20 x 10⁻⁸ |
| CuSO₄.5H₂O | 9.8 | 1 mL | 3.93 x 10⁻⁸ |
| Na₂MoO4.2H₂O | 6.3 | 1 mL | 2.60 x 10⁻⁸ |

In each system, the aeration (turbulence) and the CO₂ supply are maintained connected, thus controlling the pH.
2. After 24 hours the two systems are in conditions of feeding another two systems according to the process set forth in 1, and so on until all the remaining systems have been started up.

All the units work at atmospheric pressure and according to a reversed return system.

The production is 1060 kg/h of product with a moisture content of 10%. Said plant has a total volume of 9540 m³ distributed in 2000 modular systems arranged at three heights. The orientation of the latter is from east to west to prevent the shadow effect, the plant occupying 1.57 ha.

To achieve a production of 1060 kg/h, 2 daily extractions of 40% of the total volume in each of them are performed, according to the following extraction schedule:
- 8 hours of light → growth of the culture until the desired concentration (3g/L)
- 4 hours → extraction of 40 % of the total culture
- 8 hours of light → growth of the culture until the desired concentration (3g/L)
- 4 hours → extraction of 40 % of the total culture

The light-darkness cycle is thus the following: 8 light - 4 darkness - 8 light - 4 darkness.

The systems are located outdoors to take advantage of the natural light, it being necessary to supply artificial light (equivalent to the yearly mean of the natural light of the Mediterranean coast: 4.5 kW.h/m²/day) for the last eight hours of production. It is important to emphasize that the system has an outer sleeve in which there is a refracting and attempering liquid of the system.

The general characteristics of the culture are shown below:
- The temperature of the culture is at 25°C adjusted by the aforementioned sleeve.
- Culture salinity of 4 g/L of NaCl; this salinity is reached by working with brackish well water.
- 150 mg of commercial zeolite per liter of culture are supplied to the system, for the purpose of increasing the efficiency thereof.
- The system is fed with CO₂ from an internal combustion engine by means of pulses. The pulses begin when the pH exceeds the value of 9 and stop when this value is below 7. The gas resulting from the combustion, is treated to separate the CO₂ from the rest of the gases.
- Magnetic field. The entire culture is subjected to a continuous magnetic field intensity of 0.43T.
- The culture water which has been eliminated in the separation steps is recirculated; it is important to emphasize that the recirculated water still contains nutrients, therefore the following supply of the latter must be carried out performing a correction mainly based on the measurements of the nitrate and phosphate sensors.
- The culture is at all times subjected to turbulences caused either by bubbling (pulses of 400 Umin of air per system for 15 seconds every 30 minutes) or by the actual circulation caused by the pump of the system (8500 Uh).

### B) SEPARATION

After performing the extraction, 318 m³/h of culture are subjected to a preconcentration step by means of an electrocoagulation process. The charge of the microalgae is neutralized, leaving them in conditions of forming flocculi. The culture is then subjected to a press filtration process for the purpose of increasing the solid content. The cake which is obtained after this concentration step is a wet product with a solid concentration of 40%.

The last step that is performed is the drying step. This step is performed at a low temperature, based on a hot air convection principle, in a continuous and closed tunnel. The feed to the drying system is 2386 kg/h, the outlet of the latter being 1060 kg/h of product at a 10% moisture.

### C) TRANSFORMATION

The product with a moisture of 10% is introduced in a gasification reactor where a partial combustion of the latter is carried out at 700°C, using air as a gasifying agent. As a result, a syngas (CO₂, CO, CH₄, H₂ and N₂ mainly) is obtained with a mean calorific value of 1350 kcal/NM³.

The syngas is adjusted by means of a cooling and conditioning system so that it can then be valued in an internal combustion engine. Thus an installed electric power of 1.7 MWₑ, and a thermal power of 2.9 MWₜ are obtained. This thermal power will be used in the drying step which has been previously mentioned.

The self-consumption in relation to the electric energy produced is 19%.

### 2) PRODUCTION OF THERMAL ENERGY (FLASH PYROLYSIS FOR THERMAL ENERGY).

### A) CULTURING AND PRODUCTION

The starting point is a monospecific Pavlova lutheri culture in seawater at a concentration of 38g/L in NaCl.

To start up each of the basic plant systems, the original strain is used as the starting point, which strain is stored in small transparent containers which can be sterilized in an autoclave. The latter are stored in an incubator cooled at 4 to 12 °C, illuminated by fluorescent 8-watt (W) lamps providing a light intensity of 450 lux calculated on the surface of the culture. The objective is not to accelerate the growth but to maintain the cultures in good conditions. The cultures are not aerated nor is carbon dioxide introduced.

It is necessary to transfer the strain at monthly intervals to maintain it in a good state and vigorous. After removing the cotton wool stopper of the flask containing the strains and burning the neck of the flask with a Bunsen burner, a 20 to 50 mL inoculum is transferred to another sterile flask containing the medium previously sterilized in an autoclave. The stopper is inserted after burning the neck of the new flask.

### Steps in the production of algae

The strain (250 mL or less) continues to be isolated under light and a controlled climate (low temperature) and is only used when it is necessary to inoculate. They are neither aerated nor is carbon dioxide added. The inocula (250 mL to 4 L in volume) grow quickly for a period of 7 to 14 days at higher light intensity and temperatures with a carbon dioxide-enriched air supply. When they are ready, a small proportion of the volume is used to initiate new inocula and the main portion to start cultures on an intermediate scale (20L). Once 20L are available, the sequence is that set forth below:
- 50 L
- 250 L
- 750 L
- 2 m³
- 5 m³
- 8 m³
- 14.31 m³

The sequence going from 50L to 14.31 m³ involves a time of 3 weeks. The first photobioaccelerator into which CO₂ and air are introduced is thus started up. This photobioacceierator will allow starting up the remaining photobioaccelerators, as detailed below:
1. Half the culture passes to another system which is empty and at that same instant the necessary water is added to both. The latter, which is already attempered, comes from a "surge tank", which has previously been subjected to an ultraviolet treatment (sterilization) and the nutrient will have been added to it (the surge tank has nutrient probes to automate the nutrient supply).
The concentration per liter of culture is that set forth below:

| Component | Stock solution (gL⁻¹ dH₂O) | Amount used | Final concentration |
|---|---|---|---|
| NaNO₃ | 60 | 1 mL | 8.82 x 10⁻⁴ |
| NaH₂PO₄.H₂O | 3 | 1 mL | 3.62 x 10⁻⁵ |
| Na₂SiO₃.9H₂O | 15 | 1 mL | 1.06 x 10⁻⁴ |
| Trace metals | See below | 1 mL | --- |
| Vitamins | See below | 0.5 mL | --- |

Solution of trace metals f/2
Dissolve EDTA and the other components in 950 mL of distilled water. Take to a volume of 1 L of distilled water.

| Component | Stock solution (gL⁻¹ dH₂O) | Amount used | Final concentration |
|---|---|---|---|
| FeCl₃.6H₂O | --- | 3.15 g | 1.17 x 10⁻⁵ |
| Na₂EDTA | --- | 4.36 g | 1.17 x 10⁻⁵ |
| MnCl₂.4H₂O | 160.0 | 1 mL | 9.10 x 10⁻⁷ |
| ZnSO₄.7H₂O | 10.0 | 1 mL | 7.65 x 10⁻⁸ |
| CoCl₂.6H₂O | 5.0 | 1 mL | 4.20 x 10⁻⁸ |
| CuSO₄.5H₂O | 4.5 | 1 mL | 3.93 x 10⁻⁸ |
| Na₂MoO4.2H₂O | 3 | 1 mL | 2.60 x 10⁻⁸ |

In each system, the aeration (turbulence) and the CO₂ supply are maintained connected, thus controlling the pH.
2. After 24 hours the two systems are in conditions of feeding another two systems according to the process set forth in 1, and so on until all the remaining systems have been started up.

All the units work at atmospheric pressure.

The starting point is a plant with a daily production of 1856 kg/h of product with a moisture content of 10%. Said plant will have a total volume of 14310 m³ distributed in 1000 modular bioaccelerators arranged at three heights. The orientation of the latter will be from east to west for preventing the shadow effect, the plant occupying 2.40 ha.

To achieve a production of 1856 kg/h, 2 daily extractions of 50% of the total volume in each of them are performed, according to the following extraction schedule:
- 8 hours of light → growth of the culture until the desired concentration (3.5 g/L)
- 4 hours → extraction of 50 % of the total culture
- 8 hours of light → growth of the culture until the desired concentration (3.5 g/L)
- 4 hours → extraction of 50 % of the total culture

The light-darkness cycle is thus the following: 8 light - 4 darkness - 8 light - 4 darkness.

The systems are located outdoors to take advantage of the natural light, it being necessary to supply artificial light (equivalent to the yearly mean of the natural light of the Mediterranean coast: 4.5 kW.h/m²/day) for the last eight hours of production.

The general characteristics of the culture which is in the bioaccelerators are shown below:
- The temperature of the culture is at 27°C, adjusted with heat exchangers.
- Culture salinity of 38 g/L of NaCl; this salinity is reached by working directly with seawater.
- The system is fed with a mixture of gases (CO₂, CO, NOₓ) from the boiler by means of pulses. The pulses begin when the pH exceeds the value of 9 and stop when this value is below 7. The gas resulting from the combustion is treated to eliminate particulate material.
- 75 mg of commercial zeolite per liter of culture are supplied to the system, for the purpose of increasing the efficiency thereof.
- Magnetic field. The entire culture is subjected to a continuous magnetic field intensity of 0.50T.
- The culture water which has been eliminated in the separation steps is recirculated; it is important to emphasize that the recirculated water still contains nutrients, therefore the following supply of the latter must be carried out performing a correction mainly based on the measurements of the nitrate and phosphate sensors.
- The culture is at all times subjected to turbulences caused either by bubbling (pulses of 500 Umin of air per system for 1 minute every 30 minutes) or by the actual circulation caused by the pump of the system (7000 Uh).

### B) SEPARATION

After performing the extraction, 597 m³/h of culture are subjected to a preconcentration step by means of a coagulation-flocculation process. The coagulation is performed with aluminium sulfate, thus neutralizing the charge of the microalgae, and the flocculation is performed with a polymeric polyelectrolyte (ZETAG). The culture is then subjected to a press filtration process for the purpose of increasing the solid content. The cake which is obtained after this concentration step is a wet product with a solid concentration of 30%.

The drying with hot air is then carried out, the hot air being obtained from heating it with the outlet gases of the boiler. To that end, a Swirl Fluidizer is used, the system of which allows drying products with a very viscous behavior, using a worn screw as a feeding system.

This drying system is characterized by operating with a very short dwell time of the air and of the product. The heart of the process is the drying chamber in which the disintegration of the feed and the evaporation take place and from which the used drying air containing the dry particles flows towards the combined outlet air cleaning and product recovery system through the air/product outlet located in the upper section. The feed will be introduced approximately in the middle of the chamber which is formed by an upper cylindrical part and a lower conical part. This design leads to an internal product flow rate model which ensures a uniform drying and maintains the air disperser as a product-free area. The air disperser which contours the lower part of the drying chamber is provided with a tangential air inlet and an internal guide gate system creating the intense, controllable turbulent air flow entering the drying chamber through the bottom. The vertical rotary disintegrator, assembled with horizontal blades, is placed at the base of the air disperser. A hose filter is fixed for collecting the dry fine product and for cleaning the used drying air. The dry fine product is unloaded from the bottom of the hose filter by means of a rotary valve.

The feed to the drying system is 5567 kg/h of 70%, wet product, the outlet being 1856 kg/h at 10% moisture.

### C) TRANSFORMATION

The biomass with a moisture of 10% is introduced in a pyrolysis reactor wherein a chemical decomposition of the organic matter is carried out, caused by the heating in the absence of oxygen or other reagents, except possibly steam. A gas fraction and another solid fraction can be obtained as the result of this decomposition. The gaseous fraction in turn has a condensable fraction (a mixture of long-chain compounds: Bio-oil) and other non-condensable fraction (short-chain compounds, H₂, CH₄, C₂H₄, C₂H₆...). Given that the pyrolysis to be carried out will be at moderate temperatures (550°C) and with a residence time less than 2 seconds, the following fractions will be obtained:
- Liquid (bio-oil)→ 70% → 1623.12 kg/h
- Solid (carbon deposit) → 15% → 347.81 kg/h
- Gas → 15% → 347.81 kg/h

This pyrolysis is commonly called Flash Pyrolysis and is aimed at obtaining a liquid product.

The raw material prepared (biomass from microalgae, in this case) is used to feed a fluidized bed reactor, which is heated at 550 °C, in the absence of oxygen. The raw material evaporates in the same way as when drops of water are thrown on a hot deep fryer. The resulting gases pass through a cyclone in which the solid particles, i.e., the carbon deposit, are extracted. The gases enter a cooling tower where they are rapidly cooled using the Bio-oil already obtained from the process.

The Bio-oil condenses and falls inside the tank of the product, whereas the non-condensable gases are again sent to the reactor to maintain the temperature of the process.

100% of the raw material is used in the process for producing bio-oil and carbon deposit. Given that the non-condensable gases are used as energy for feeding the process, nothing is wasted and waste is not produced. The non-condensed inflammable gases are recirculated to provide 75% of the energy required by the pyrolysis process.

Once the liquid, which has a calorific value of 7500 kcal/kg, is obtained, it is passed to a boiler which allows obtaining a thermal power of 11.3 kw_{th.}

### 3) PRODUCTION OF ELECTRIC ENERGY

### (Solid boiler + Steam turbine)

### A) CULTURING AND PRODUCTION

The starting point is a multispecific Pavlova lutheri and Tetraselmis sp. culture in seawater at a concentration of 32g/L in NaCl.

To start up each of the basic plant systems, original strains are used as the starting point, which strains are stored in small transparent containers which can be sterilized in an autoclave. The latter are stored in an incubator cooled at 4 to 12°C, illuminated by fluorescent 8-watt (W) lamps providing a light intensity of 450 lux calculated on the surface of the culture. The objective is not to accelerate the growth but to maintain the cultures in good conditions. The cultures are not aerated nor is carbon dioxide introduced.

It is necessary to transfer the strains at monthly intervals to maintain them in a good state and vigorous. After removing the cotton wool stopper of the flasks containing the strains and burning their neck with a Bunsen burner, a 20 to 50 mL inoculum of each of the two species is transferred to another sterile flask containing the medium previously sterilized in an autoclave. The stopper is inserted after burning the neck of the new flask.

### Steps in the production of algae

The strains (250 mL or less) will continue to be isolated under light and a controlled climate (low temperature) and will only be used when it is necessary to inoculate. They are neither aerated nor is carbon dioxide added. The inocula (250 mL to 4 L in volume) grow quickly for a period of 7 to 14 days at higher light intensity and temperatures with a carbon dioxide-enriched air supply. When they are ready, a small proportion of the volume of each of the fermenters (each fermenter with a different species) is used for mixing the three species in order to take the next step on an intermediate scale (20L). Once this culture is available, the sequence is that set forth below:
- 50 L
- 250 L
- 750 L
- 2 m³
- 5 m³
- 15m³
- 19.08 m³

The sequence going from 50L up to 19.08 m³ involves a time of 4 weeks. The first system into which CO₂ and air are injected is thus started up. This system allows starting up the remaining systems, as is detailed below:
1. Half the culture passes to another system which is empty and at that same instant, the necessary water is added to both. The latter, which is already attempered, comes from a "surge tank", which has previously been subjected to an ultraviolet treatment (sterilization) and the nutrient will have been added to it (the surge tank has nutrient probes to automate the nutrient supply).
The concentration per liter of culture is that set forth below:

| *Component* | *Stock solution (gL⁻¹ dH₂O)* | *Amount used* | *Final concentration* |
|---|---|---|---|
| NaNO₃ | 60 | 1 mL | 8.82 x 10⁻⁴ |
| NaH₂PO₄.H₂O | 4 | 1 mL | 3.62 x 10⁻⁵ |
| Na₂SiO₃.9H₂O | 25 | 1 mL | 1.06 x 10⁻⁴ |
| Trace metals | See below | 1 mL | --- |
| Vitamins | See below | 0.5 mL | --- |

Solution of trace metals f/2
Dissolve EDTA and the other components in 950 mL of distilled water. Take to a volume of 1 L of distilled water.

| *Component* | *Stock solution (gL⁻¹ dH₂O)* | *Amount* used | *Final concentration* |
|---|---|---|---|
| FeCl₃.6H₂O | --- | 3.15 g | 1.17 x 10⁻⁵ |
| Na₂EDTA | --- | 4.36 g | 1.17 x 10⁻⁵ |
| MnCl₂.4H₂O | 160.0 | 1 mL | 9.10 x 10⁻⁷ |
| ZnSO₄.7H₂O | 18.0 | 1 mL | 7.65 x 10⁻⁸ |
| CoCl₂.6H₂O | 10.0 | 1 mL | 4.20 x 10⁻⁸ |
| CuSO₄.5H₂O | 11.1 | 1 mL | 3.93 x 10⁻⁸ |
| Na₂MoO4.2H₂O | 6.3 | 1 mL | 2.60 x 10⁻⁸ |

In each system, the aeration (turbulence) and the CO₂ supply are maintained connected, thus controlling the pH.
2. After 24 hours the two systems are in conditions of feeding another two systems according to the process set forth in 1, and so on until all the remaining systems have been started up.

All the units work at atmospheric pressure.

The starting point is a plant with a production of 1011 kg/h of product with a moisture content of 15%. Said plant has a total volume of 14316 m³ distributed in 750 modular bioaccelerators arranged at three heights. The orientation of these bioaccelerators is from east to west in order to minimize the shadow effect, the plant occupying 2.40 ha.

To achieve a production of 1011 kg/h, a daily extraction of 60% of the total volume of the plant is performed, according to the following schedule:
- 16 hours of light → growth of the culture until the desired concentration (2.4 g/L)
- 8 hours → extraction of 60% of the total culture

The light-darkness cycle is thus the following:
16 hours of light - 8 hours of darkness.

The systems are located outdoors, sunlight being the main light source. An artificial light supply (equivalent to 45% of the yearly mean of the natural light of the Mediterranean coast, i.e. 2.0 kWh/m²/day) is necessary for the first two hours and the last six hours of production.

The general characteristics of the culture are shown below:
- The temperature of the culture ranges between 18 and 23°C during the production step.
- Culture salinity of 32 g/L of NaCl; this salinity is reached by working directly with seawater.
- 50 mg of commercial zeolite per liter of culture are supplied to the system, for the purpose of increasing the efficiency thereof.
- CO₂ from the combustion gases of the solid boiler is fed. For the purpose of storing the CO₂, it is passed through a countercurrent absorption column with an NaOH solution in the water which is recirculated, in order to obtain carbonate and bicarbonate in liquid phase. The feed is carried out continuously.
- The culture water which has been eliminated in the separation steps is recirculated; it is important to emphasize that the recirculated water still contains nutrients, therefore the following supply of the latter must be carried out performing a correction mainly based on the measurements of the nitrate and phosphate sensors.
- The culture is at all times subjected to turbulences caused either by bubbling (pulses of 100 Umin of air per system for 30 seconds every 20 minutes) or by the actual circulation caused by the pump of the system (3000 Uh).

### B) SEPARATION

After performing the extraction, 358 m³/h of culture are subjected to a preconcentration step by means of sand filters. Once the filters are saturated, there were washed in countercurrent to recover the microalgae. Freshwater with a flow rate of 18 m³/h is used. The concentrated culture (4.8% in solids) is subjected to a centrifugation process, using a vertical plate centrifuge. A concentrated culture is obtained, where the solid content increased to 13%.

The last step that is performed is the drying step. It is carried out by means of a spray-dryer. The culture is circulated in countercurrent with hot air inside a sprayer.

The feed to the drying system is 6607 kg/h, the outlet of the later being 1011 kg/h of product at 15% moisture.

### C) TRANSFORMATION

The product with a moisture of 15% is subjected to a combustion process in a water-tube solid boiler with a mobile grate. The vapor generated, 7016 kg/h (40 bar and 350°C), is introduced in a steam turbine which allows obtaining an electric power of 1.4 MW.

Self-consumption with respect to the electric energy produced is 24%.

### 4) PRODUCTION OF BIODIESEL

### (FATTY ACID TRANSESTERIFICATION-ESTERIFICATION)

### A) CULTURING AND PRODUCTION

The starting point is a multispecific Isochrysis galbana, Phaedoactylum tricornutum and Tetraselmis sp. culture in seawater at a concentration of 35-38g/L in NaCl.

To start up the system, original strains are used as the starting point, which strains are stored in small transparent containers which can be sterilized in an autoclave. The latter are stored in an incubator cooled at 4 to 12°C, illuminated by fluorescent 8-watt (W) lamps providing a light intensity of 450 lux calculated on the surface of the culture. The objective is not to accelerate the growth but to maintain the cultures in good conditions. The cultures are not aerated nor is carbon dioxide introduced.

It is necessary to transfer the strains at monthly intervals to maintain them in a good state and vigorous. After removing the cotton wool stopper of the flasks containing the strains and burning their neck with a Bunsen burner, a 20 to 50 mL inoculum of each of the two species is transferred to another sterile flask containing the medium previously sterilized in an autoclave. The stopper is inserted after burning the neck of the new flask.

### Steps in the production of algae

The strains (250 mL or less) will continue to be isolated under light and a controlled climate (low temperature) and will only be used when it is necessary to inoculate. They are neither aerated nor is carbon dioxide added. The inocula (250 mL to 4 L in volume) grow quickly for a period of 7 to 14 days at higher light intensity and temperatures with a carbon dioxide-enriched air supply. When they are ready, a small proportion of the volume of each of the fermenters (each fermenter with a different species) is used for mixing the three species in order to take the next step on an intermediate scale (20L). Once this culture is available, the sequence is that set forth below:
- 50 L
- 250 L
- 750 L
- 2 m³
- 4.77 m³
- 9.54 m³ (volume of the system)

The sequence going from 50L up to 9.54 m³ involves a time of 2 weeks. The first system into which CO₂ and air are injected is thus started up. This system allows starting up the remaining systems, as is detailed below:
1. Half the culture passes to another system which is empty and at that same instant, the necessary water is added to both. The latter, which is already attempered, comes from a "surge tank", which has previously been subjected to an ultraviolet treatment (sterilization) and the nutrient will have been added to it (the surge tank has nutrient probes to automate the nutrient supply).
The concentration per liter of culture is that set forth below:

| Component | Stock solution (gL⁻¹ dH₂O) | Amount used | Final concentration |
|---|---|---|---|
| NaNO₃ | 50 | 1 mL | 8.82 x 10⁻⁴ |
| NaH₂PO₄.H₂O | 7 | 1 mL | 3.62 x 10⁻⁵ |
| Na₂SiO₃.9H₂O | 10 | 1 mL | 1.06 x 10⁻⁴ |
| Trace metals | See below | 1 mL | --- |
| Vitamins | See below | 0.5 mL | --- |

Solution of trace metals f/2
Dissolve EDTA and the other components in 950 mL of distilled water. Take to a volume of 1 L of distilled water.

| *Component* | *Stock solution (gL⁻¹ dH₂O)* | *Amount used* | *Final concentration* |
|---|---|---|---|
| FeCl₃.6H₂O | --- | 3.15 g | 1.17 x 10⁻⁵ |
| Na₂EDTA | --- | 4.36 g | 1.17 x 10⁻⁵ |
| MnCl₂.4H₂O | 80.0 | 1 mL | 9.10 x 10⁻⁷ |
| ZnSO₄.7H₂O | 10.0 | 1 mL | 7.65 x 10⁻⁸ |
| CoCl₂.6H₂O | 5.0 | 1 mL | 4.20 x 10⁻⁸ |
| CuSO₄.5H₂O | 4.5 | 1 mL | 3.93 x 10⁻⁸ |
| Na₂MoO4.2H₂O | 6.5 | 1 mL | 2.60 x 10⁻⁸ |

In each system, the aeration (turbulence) and the CO₂ supply are maintained connected, thus controlling the pH.
2. After 24 hours the two systems are in conditions of feeding another two systems according to the process set forth in 1, and so on until all the remaining systems have been started up.

All the units work at atmospheric pressure and according to a reversed return system.

The starting point is a plant with a production of 2510 kg/h of product with a moisture content of 5%. Said plant has a total volume of 19080 m³ distributed in 2000 modular bioaccelerators arranged at three heights. The orientation of the latter will be from east to west in order to minimize the shadow effect, the plant occupying 3.2 ha.

To achieve a production of 2510 kg/h, 2 daily extractions of 50% of the total volume in each of them are performed, according to the following extraction schedule:
• 8 hours of light → growth of the culture until the desired concentration (3 g/L)
• 4 hours → extraction of the 50 % of the total culture
• 8 hours of light → growth of the culture until the desired concentration (3 g/L)
• 4 hours → extraction of the 50 % of the total culture

The light-darkness cycle is thus the following: 8 light - 4 darkness - 8 light - 4 darkness.

The systems are located outdoors to take advantage of the natural light, it being necessary to supply artificial light (equivalent to the yearly mean of the natural light of the Mediterranean coast: 4.5 kW.h/m²/day) for the last eight hours of production.

The general characteristics of the culture are shown below:
- The temperature of the culture is at 25°C, adjusted with heat exchangers.
- Culture salinity of 35-38 g/L of NaCl; this salinity is reached by working directly with seawater.
- CO₂ is fed to the system by pulses; this CO₂ comes from the internal combustion engine when the pH is above 9, and for when this value is below 7.

The gas resulting from the combustion is treated to separate the CO₂ from the rest of the gases.
- 200 mg of commercial zeolite per liter of culture are supplied to the system, for the purpose of increasing the efficiency thereof.
- Magnetic field. The entire culture is subjected to a continuous magnetic field intensity of 0.50 T.
- The culture water which has been eliminated in the separation steps is recirculated; it is important to emphasize that the recirculated water still contains nutrients, therefore the following supply of the latter must be carried out performing a correction mainly based on the measurements of the nitrate and phosphate sensors.
- The culture is at all times subjected to turbulences caused either by bubbling (pulses of 400 Umin of air per system for 30 seconds every 10 minutes) or by the actual circulation caused by the pump of the system (8500 Uh per system).

### B) SEPARATION

After performing the extraction, 795 m³/h of culture are subjected to a preconcentration step by means of a tangential filtration process. This system consists of a filtration in which a filtering medium, usually a polymeric membrane allows dividing a solid and fluid stream, into another clean (permeated) fluid stream and a concentrated mixture. According to thus, the concentrate liquid obtained has a solid content of 6%.

This liquid concentrated to 6% passes to a chamber where it is preheated at 60°C through a heat exchanger. A compressor generates a vacuum in said chamber such that the vapor is suctioned by the compressor and sent to a water condensation chamber, this water being returned to the system. Said device is known as an MVR (Mechanical Vapor Recompression) device, thus obtaining a concentrate of 15% in solids.

This concentrate is then fed to a multiple-effect evaporator system which allows obtaining 2510 kg/h of product at a 5% moisture.

### C) TRANSFORMATION

Out of the 2510 kg/h of 5% product, only 40% with respect to the dry matter, 954 kg/h, can be transesterified. To prevent the use of catalysts which can give rise to secondary reactions which lower the overall yield of the methylation process, an esterification-transesterification is carried in supercritical conditions; (high pressure and temperature conditions). Thus, 906 kg/h of a mixture of methyl esters (using methanol) which have physicochemical characteristics very similar to the diesel used in automobiles is obtained according to a 95% conversion.

Once the esters are available, they are stabilized and ready for their sale.

The installed power necessary for carrying out the entire process is 2.7 MWₑ.

### 5) PRODUCTION OF ELECTRIC ENERGY

### (GASIFICATION BY PLASMA + COMBINED CYCLES

### A) CULTURING AND PRODUCTION

The starting point is a monospecific skeletonema costatum culture in seawater at a concentration of 35g/L in NaCl.

To start up the systems, an original strain is used as the starting point, which strain is stored in small transparent containers which can be sterilized in an autoclave.
The latter are stored in an incubator cooled at 4 to 12°C, illuminated by fluorescent 8-watt (W) lamps providing a light intensity of 450 lux calculated on the surface of the culture. The objective is not to accelerate the growth but to maintain the cultures in good conditions. The cultures are not aerated nor is carbon dioxide introduced.

It is necessary to transfer the strains at monthly intervals to maintain them in a good state and vigorous. After removing the cotton wool stopper of the flasks containing the strains and burning their neck with a Bunsen burner, a 20 to 50 mL inoculum of each of the two species is transferred to another sterile flask containing the medium previously sterilized in an autoclave. The stopper is inserted after burning the neck of the new flask.

### Steps in the production of algae

The strain (250 mL or less) will continue to be isolated under light and a controlled climate (low temperature) and is only used when it is necessary to inoculate.
They are neither aerated nor is carbon dioxide added. The inocula (250 mL to 4 L in volume) grow quickly for a period of 7 to 14 days at higher light intensity and temperatures with a carbon dioxide-enriched air supply. When they are ready, a small proportion of the volume of each of the fermenters (each fermenter with a different species) is used for initiating new inocula and main portion is use to start cultures on an intermediate scale (20L). Once 20 L are available, the sequence is that set forth below:
• 50 L
• 250 L
• 750 L
• 2 m³
• 4.77 m³

The sequence going from 50L up to 4.77 m³ involves a time of 2 weeks. The first system into which CO₂ and air are injected is thus started up. This system allows starting up the remaining systems, as is detailed below:
1. Half the culture passes to another system which is empty and at that same instant, the necessary water is added to both. The latter, which is already attempered, comes from a "surge tank", which has previously been subjected to an ultraviolet treatment (sterilization) and the nutrient will have been added to it (the surge tank has nutrient probes to automate the nutrient suppiy).
The concentration per liter of culture is that set forth below:

| *Component* | *Stock solution (gL*⁻¹ *dH₂O)* | *Amount used* | *Final concentration* |
|---|---|---|---|
| NaNO₃ | 150 | 1 mL | 8.82 x 10⁻⁴ |
| NaH₂PO₄.H₂O | 4 | 1 mL | 3.62 x 10⁻⁵ |
| Na₂SiO₃.9H₂O | 8 | 1 mL | 1.06 x 10⁻⁴ |
| Trace metals | See below | 1 mL | --- |
| Vitamins | See below | 0.5 mL | --- |

Solution of trace metals f/2
Dissolve EDTA and the other components in 950 mL of distilled water. Take to a volume of 1 L of distilled water.

| *Component* | *Stock solution (gL⁻¹ dH₂O)* | *Amount used* | *Final concentration* |
|---|---|---|---|
| FeCl₃.6H₂O | --- | 3.15 g | 1.17 x 10⁻⁵ |
| Na₂EDTA | --- | 4.36 g | 1.17 x 10⁻⁵ |
| MnCl₂.4H₂O | 15.0 | 1 mL | 9.10 x 10⁻⁷ |
| ZnSO₄.7H₂O | 25.0 | 1 mL | 7.65 x 10⁻⁸ |
| CoCl₂.6H₂O | 15.0 | 1 mL | 4.20 x 10⁻⁸ |
| CuSO₄.5H₂O | 4.5 | 1 mL | 3.93 x 10⁻⁸ |
| Na₂MoO4.2H₂O | 6.5 | 1 mL | 2.60 x 10⁻⁸ |

In each system, the aeration (turbulence) and the CO₂ supply are maintained connected, thus controlling the pH.
2. After 24 hours the two systems are in conditions of feeding another two systems according to the process set forth in 1, and so on until all the remaining systems have been started up.

All the units work at atmospheric pressure and according to a reversed return system.

The starting point is a plant with a production of 9942 kg/h of end product with a moisture of 20%. Said plant has a total volume of 47721 m³ distributed in 10000 modular systems arranged at three heights. The orientation of the latter is from east to west in order to minimize the shadow effect, the plant occupying 6 ha.

To achieve a production of 9942 kg/h, three daily extractions of 33% of the total volume of the plant are performed, according to the following schedule:
• 7 hours of light → growth of the culture until the desired concentration (4 g/L)
• 1 hour → extraction of 33 % of the total culture
• 7 hours of light → growth of the culture until the desired concentration (4 g/L)
• 1 hour→ extraction of 33 % of the total culture
• 7 hours of light → growth of the culture until the desired concentration (4 g/L)
• 1 hour → extraction of 33 % of the total culture

The light-darkness cycle is thus the following:
7 hours of light - 1 hour of darkness - 7 hours of light - 1 hour of darkness - 7 hours of light - 1 hour of darkness.

The systems are located outdoors, the main light source being from the sun. An artificial light supply (equivalent to 45% of the yearly mean of the natural light of the Mediterranean coast, i.e. 4.5 kWh/m²/day), is necessary for the first two hours and the last nine hours of production.

The start-up of one of the systems responds to the following systematic:
• Half of each system is seeded with a culture the concentration of which is exactly that of extraction (4 g/L).
• The other half is filled with seawater, to which the nutrients have been previously added.
• The start-up is considered to be ended after 48 hours and the aforementioned extraction schedule starts from this same moment.

The general characteristics of the culture are shown below:
- The temperature of the culture is 21 °C, adjusted with heat exchangers.
- Culture salinity of the culture of 35 g/L of NaCl; this salinity is reached working directly with brackish well water.
- The amount of CO₂ necessary for the production of the plant is introduced in the culture medium by means of an adsorption process. The mixture of gases from the plasma gasification is circulated in countercurrent with the NaOH-rich culture medium, such that the CO₂ present reacts with the sodium hydroxide to form sodium bicarbonate and carbonate, the latter being the carbon source necessary for production.
- 125 mg of commercial zeolite per liter of culture are supplied to the system, for the purpose of increasing the efficiency thereof.
- Magnetic field. The entire culture is subjected to a variable magnetic field intensity of 20 mT.
- The culture water which has been eliminated in the separation steps is recirculated; it is important to emphasize that the recirculated water still contains nutrients, therefore the following supply of the latter must be carried out performing a correction mainly based on the measurements of the nitrate and phosphate sensors.
- The culture is at all times subjected to turbulences caused either by bubbling (pulses of 250 Umin of air per bioaccelerator for 60 seconds every 40 minutes) or by the actual circulation caused by the pump of the system (9000 Uh per system).

### B) SEPARATION

After performing the extraction, 1989 m³/h of culture are subjected to a preconcentration step by means of tangential filtration, achieving a solid concentration of 8%. The concentrated culture is then subjected to a centrifugation process, using a special vertical axis centrifuge for the treatment of phytoplankton.

Finally, the biomass obtained (35% in solids) is dried using a straight turbodryer. The feed to the turbodryer is 22724 kg/h plus 40% of dry product.

### C) TRANSFORMATION

The product, 9942 kg/h, with a moisture of 20% is subjected to a plasma gasification process, the transformation of the product being carried out in a syngas. This gasification takes place in an oxygen-poor atmosphere using plasma (superheated and ionized air) as a heat source. The generated syngas from the gasification reactor is cooled and cleaned, eliminating the sulfated, chlorinated compounds, volatile metals and any particle. The gas then feeds a gas turbine to produce electricity in a combined cycle. The installed electric power is 30 MWₑ.

The self-consumption with respect to the electric energy produced is 11%.

### 6) OBTAINING A HIGH NUTRITIONAL VALUE PRODUCT

### A) CULTURING AND PRODUCTION

The starting point is a monospecific spirulina maxima culture, in brackish well water with a concentration 4g/L in NaCl.

To start up each of the basic units of the plant, the original strain is used as the starting point, which strain is stored in small transparent containers which can be sterilized in an autoclave. The latter are stored in an incubator cooled at 4 to 12°C, illuminated by fluorescent 8-watt (W) lamps providing a light intensity of 450 lux calculated on the surface of the culture. The objective is not to accelerate the growth but to maintain the cultures in good conditions. The cultures are not aerated nor is carbon dioxide introduced.

It is necessary to transfer the strains at monthly intervals to maintain them in a good state and vigorous. After removing the cotton wool stopper of the flasks containing the strains and burning their neck with a Bunsen burner, a 20 to 50 mL inoculum is transferred to another sterile flask containing the medium previously sterilized in an autoclave. The stopper is inserted after burning the neck of the new flask.

### Steps in the production of algae

The strain (250 mL or less) continues to be isolated under light and a controlled climate (low temperature) and is only used when it is necessary to inoculate. They are neither aerated nor is carbon dioxide added. The inocula (250 mL to 4 L in volume) grow quickly for a period of 7 to 14 days at higher light intensity and temperatures with a carbon dioxide-enriched air supply. When they are ready, a small proportion of the volume is used for initiating new inocula and main portion is used to start cultures on an intermediate scale (20L). Once 20 L are available, the sequence is that set forth below:
• 50 L
• 250 L
• 750 L
• 2 m³

The sequence going from 50L up to 2 m³ involves a time of 2 weeks. The first system into which CO₂ and air is injected is thus started up. This system allows starting up the remaining systems, as is detailed below:
1. Half the culture passes to another system which is empty and at that same instant, the necessary water is added to both. The latter, which is already attempered, comes from a "surge tank", which has previously been subjected to an ultraviolet treatment (sterilization) and the nutrient will have been added to it (the surge tank has nutrient probes to automate the nutrient supply).
The concentration per liter of culture is that set forth below:

| Component | Stock solution (gL⁻¹ dH₂O) | Amount used | Final concentration |
|---|---|---|---|
| NaNO₃ | 75 | 1 mL | 8.82 x 10⁻⁴ |
| NaH₂PO₄.H₂O | 5 | 1 mL | 3.62 x 10⁻⁵ |
| Na₂SiO₃.9H₂O | 30 | 1 mL | 1.06 x 10⁻⁴ |
| Trace metals | See below | 1 mL | --- |
| Vitamins | See below | 0.5 mL | --- |

Solution of trace metals f/2
Dissolve EDTA and the other components in 950 mL of distilled water. Take to a volume of 1 L of distilled water

| Component | Stock solution (gL⁻¹ dH₂O) | Amount used | Final concentration |
|---|---|---|---|
| FeCl₃.6H₂O | --- | 3.15 g | 1.17 x 10⁻⁵ |
| Na₂EDTA | --- | 4.36 g | 1.17 x 10⁻⁵ |
| MnCl₂.4H₂O | 180.0 | 1 mL | 9.10 x 10⁻⁷ |
| ZnSO₄.7H₂O | 22.0 | 1 mL | 7.65 x 10⁻⁸ |
| CoCl₂.6H₂O | 10.0 | 1 mL | 4.20 x 10⁻⁸ |
| CuSO₄.5H₂O | 9.8 | 1 mL | 3.93 x 10⁻⁸ |
| Na₂MoO4.2H₂O | 6.3 | 1 mL | 2.60 x 10⁻⁸ |

In each system, the aeration (turbulence) and the CO₂ supply are maintained connected, thus controlling the pH.
2. After 24 hours the two photobioaccelerators are in conditions of feeding another two photobioaccelerators according to the process set forth in 1, and so on until all the photobioaccelerators have been started up.

All the units work at atmospheric pressure and according to a reversed return system.

The production will be 333 kg/h of end product. Said plant will have a total volume of 2000 m³ distributed in 1000 modular systems arranged at two heights. The orientation of the latter will be east to west in order to prevent the shadow effect, the plant occupying 2.3 ha.

To achieve a production of 333 kg/h, 2 daily extractions of 50% of the total volume in each of them are performed, according to the following extraction schedule:
• 8 hours of light → growth of the culture until the desired concentration (4g/L)
• 4 hours → extraction of 50 % of the total culture
• 8 hours of light → growth of the culture until the desired concentration (4g/L)
• 4 hours extraction of 50 % of the total culture

The light-darkness cycle is thus the following: 8 light - 4 darkness - 8 light - 4 darkness.

The systems are located outdoors to take advantage of the natural light, it being necessary to supply artificial light (equivalent to the yearly mean of the natural light of the Mediterranean coast: 4.5 kW.h/m²/day) for the last eight hours of production. It is important to emphasize that the system has an outer sleeve in which there is a refracting and attempering liquid of the system.

The general characteristics of the culture are detailed below:
- The temperature of the culture is at 25°C, adjusted with the aforementioned sleeve.
- Culture salinity of 4 g/L of NaCl; this salinity is reached by working directly with seawater.
- CO₂ is fed to the system by pulses; this CO₂ comes from the internal combustion engine when the pH is above 9, and for when this value is below 7.

The gas resulting from the combustion is treated to separate the CO₂ from the rest of the gases.
- The culture water which has been eliminated in the separation steps is recirculated; it is important to emphasize that the recirculated water still contains nutrients, therefore the following supply of the latter must be carried out performing a correction mainly based on the measurements of the nitrate and phosphate sensors.
- The culture is at all times subjected to turbulences caused either by bubbling (pulses of 600 Umin of air per system for 20 seconds every 20 minutes) or by the actual circulation caused by the pump of the system (9500 Uh).

### B) SEPARATION

After performing the extraction, 42 m³/h of culture are subjected to a concentration step by means of a centrifugation process. A concentrate with a solid content of 17% is obtained.

The last step which is performed is the drying step. This step is performed by means of a lyophilization process. The feed to the drying system is 1959 kg/h, to obtain a production of 333 kg/h with the following composition:
- High biological value proteins: 71.1% by weight in relation to the dry product;
- Carbohydrates: 16.5% by weight in relation to the dry product;
- Vitamins: 1.8% by weight in relation to the dry product;
- Minerals and trace elements: 3.6% by weight in relation to the dry product;
- Fats: 6.7% by weight in relation to the dry product;
- Others: 0.3% by weight in relation, to the dry product.

## Claims

1. A process for obtaining a high nutritional value product and/or for transforming it into energy resources, **characterized in that** it comprises the following steps:
a. culturing of photosynthetic species with reversed return;
b. catalytic production;
c. emptying; and
d. separation.

2. The process according to claim 1, **characterized in that** the culture is monospecific or multispecific.

3. The process according to claim 1, **characterized in that** the culturing of photosynthetic species comprises the steps of:
a. inoculating the aquatic photosynthetic strains;
b. adding the culture medium;
c. sterilizing;
d. adding nutrients;
e. adjusting the temperature;
f. adjusting the salinity;
g. adjusting the pH;
h. adjusting the pressure; and
i. generating turbulences.

4. The process according to claim 3, **characterized in that** the liquid medium is selected from the group consisting of freshwater, salt water, brackish water or waste water from urban waste, previously treated with ultraviolet and filtration.

5. The process according to claim 3, **characterized in that** the sterilization is carried by means of antibiotics fungicides.

6. The process according to claim 3, **characterized in that** the nutrients are supplied in an exogenous or endogenous manner or a combination thereof and selected from the group consisting of:
a) compounds with a carbon supply;
b) compounds with a nitrogen supply;
c) compounds with a hydrogen supply;
d) compounds with a phosphorus supply; and
e) compounds with an oxygen supply.

7. The process according to claim 1, **characterized in that** the catalytic production step comprises the following steps:
a. stirring the culture medium;
b. attempering;
c. altering metabolic pathways and accelerating cell proliferation;
d. catalyzing; and
e. illuminating.

8. The process according to claim 7, **characterized in that** the stirring step is by pulses with culture circulation by injection of air, N₂, CO₂, CO, NOx, gases from a combustion or a combination thereof.

9. The process according to claim 7, **characterized in that** the attemperation is carried out by means of heat exchangers or submerging the feed underground or by means of adding cold or hot air or by means of sleeves.

10. The process according to claim 7, **characterized in that** the alteration of metabolic pathways and the acceleration of cell proliferation occur by applying a variable and/or constant intensity magnetic field.

11. The process according to claim 7, **characterized in that** the catalyzing step is carried out by means of a magneto-hydro-photosynthetic catalyst and/or natural or artificial zeolite-type catalysts.

12. The process according to claim 7, **characterized in that** the illumination is carried out in a natural or artificial manner or a combination thereof by light cycles of 6 to 24 hours and darkness cycles of 0 to 18 hours.

13. The process according to claim 1, **characterized in that** the production step is carried out by cycles of 6 to 120 hours, preferably of 8 to 12 hours.

14. The process according to claim 1, **characterized in that** in the emptying step from 10 to 100% of the culture is extracted per day. _

15. The process according to claim 1 **characterized in that** the separation step comprises the following steps:
a. preconcentration;
b. concentration; and
c. drying.

16. The process according to claim 15, **characterized in that** the preconcentration is carried out by means of techniques selected from the group consisting of filtration, flocculation, electrocoagulation, ultrasound, mechanical vapor recompression, centrifugation, decantation or a combination thereof.

17. The process according to claim 15, **characterized in that** the concentration is carried out by means of techniques selected from the group consisting of centrifugation, mechanical vapor recompression, decantation, press filter or a combination thereof.

18. The process according to claim 15, **characterized in that** the drying is carried out by means of techniques selected from the group consisting of low-temperature thermal drying, turbodryer, spray dryer, lyophilization, multiple effect evaporator, air drying or a combination thereof.

19. The process according to claim 1, **characterized in that** after the separation step a transformation step for transforming the product obtained in the previous step is carried out.

20. The process according to claim 19, **characterized in that** the transformation is carried out by means of gasification.

21. The process according to claim 19, **characterized in that** the transformation is carried out by means of pyrolysis.

22. The process according to claim 19, **characterized in that** the transformation is carried out by means of thermochemical liquefaction.

23. The process according to claim 19, **characterized in that** the transformation is carried out by means of a hydrogen production process.

24. The process according to claim 19, **characterized in that** the transformation is carried out by means of extraction and transesterification-esterification.

25. The process according to claim 19, **characterized in that** the transformation is carried out by means of anaerobic digestion.

26. The process according to claim 19, **characterized in that** the transformation is carried out by means of direct combustion.

27. The process according to claim 19, **characterized in that** a feedback step is carried out in the separation step and/or after the transformation step.

28. A concentrate of photosynthetic strains obtained according to the process of claims 1 to 18, **characterized in that** it comprises the following elements:
- high biological value proteins;
- carbohydrates;
- B-group vitamins;
- minerals and trace elements;
- fats; and
- others.

29. The concentrate of photosynthetic strains according to claim 28;
**characterized in that** the high energy value proteins are in a ratio from 40% to 85% by weight, the carbohydrates are in a ratio from 10% to 50% by weight, the B-group vitamins from 0% to 2% by weight, the minerals and trace elements from 0% to 6% by weight, the fats from 1% to 10% by weight and others from 0% to 0.5% by weight in relation to the dry product.

30. A use of the concentrate of photosynthetic strains of claims 28 and 29 as a high nutritional value product.

31. A use of the concentrate of photosynthetic strains of claims 28 and 29 as a product for its transformation into electric energy, into thermal energy, fuels and/or synthesis gas.
